Europäisches Patentamt

(19) European Patent Office

Office européen des brevets

(11) Numéro de publication: **0 172 596**
**B1**

(12) **FASCICULE DE BREVET EUROPÉEN**

(45) Date de publication du fascicule du brevet:
03.02.88

(21) Numéro de dépôt: **85201298.8**

(22) Date de dépôt: **09.08.85**

(51) Int. Cl.⁴: **C 07 C 17/34,** B 01 J 31/02

(54) Procédé de déshydrochloration pyrolytique d'halogénoalcanes en présence d'un initiateur à base de produit chloré et initiateur utilisé dans un tel procédé.

(30) Priorité: **20.08.84 FR 8413050**

(43) Date de publication de la demande:
**26.02.86 Bulletin 86/9**

(45) Mention de la délivrance du brevet:
**03.02.88 Bulletin 88/5**

(84) Etats contractants désignés:
**AT BE CH DE FR GB IT LI LU NL SE**

(56) Documents cité:
**DE-B-1 210 800**
**FR-A-947 324**

(73) Titulaire: **SOLVAY & Cie (Société Anonyme), Rue du Prince Albert, 33, B-1050 Bruxelles (BE)**

(72) Inventeur: **Franklin, James, Rue Edouard Olivier, 28, B-1170 Bruxelles (BE)**

EP 0 172 596 B1

## Description

La présente invention concerne un procédé de déshydrochloration pyrolytique d'halogénoalcanes en présence d'un initiateur à base de produit chloré ainsi qu'un initiateur pour la déshydrochloration pyrolytique d'halogénoalcanes utilisé dans un tel procédé.

Il est connu par le brevet français 947 324 que la déshydrochloration pyrolytique d'halogénoalcanes, notamment du dichloréthane, du tétrachloréthane, du 1,1,2-trichloréthane et du dichloropropane, peut être catalysée ou initiée par adjonction de petites quantités de chlore ou d'une substance fournissant du chlore à une température élevée, par exemple de l'hexachloréthane.

Il est également connu par le document DE-B-1 210 800 de fabriquer du chlorure de vinyle par déshydrochloration pyrolytique de dichloréthane, à 500-620° C sous une pression absolue de 3-20 atmosphères (3,06-20,4 bars) en présence de 0,5-2 % en poids d'un initiateur constitué par un hydrocarbure chloré tel que l'hexachloréthane, le tétrachlorure de carbone ou le perchlorethylène, à l'exclusion d'hydrocarbures chlorés comportant plus de deux atomes de carbone par molécule afin d'éviter la formation de sous-produits.

Ces procédés connus présentent certains inconvénients. Le chlore réagit quasi-intégralement avec le composé à déshydrochlorer, ce qui conduit à la formation de sous-produits indésirés et abaisse la sélectivité. Ainsi dans le cas de la déshydrochloration pyrolytique du 1,2-dichloréthane, le chlore introduit comme initiateur transforme le dichloréthane essentiellement en 1,1,2-trichloréthane et en 1,1- et 1,2-dichloréthylènes. Par ailleurs, les hydrocarbures chlorés connus comme initiateurs ne conduisent, en deça de 350°C, qu'à des taux de pyrolyse (=taux de transformation du halogénoalcane en composé insaturé correspondant) peu élevés. Pour augmenter ces taux de pyrolyse, on est amené à travailler à haute température, ce qui augmente en conséquence les dépenses d'energie et donne lieu à la formation de sous-produits et de coke. Ce dernier se dépose sur les parois du réacteur de pyrolyse, nécessitant ainsi des arrêts périodiques pour son nettoyage.

La présente invention a pour but de remédier à ces inconvénients en fournissant un nouveau procédé pour la déshydrochloration pyrolytique d'halogénoalcanes en présence d'un nouvel initiateur capable de fonctionner à des températures plus modérées. En outre, la présente invention permet de valoriser l'hexachlorobutadiène-1,3 qui constitue un sous-produit toxique et dépourvu pratiquement de débouchés, qui devait être jusqu'ici détruit par brûlage.

L'invention concerne à cet effet un procédé de déshydrochloration pyrolytique d'halogénoalcanes en présence d'un initiateur à base de produit chloré qui comprend principalement du décachlorobutane ou un octachlorobutène tel que l'octachlorobutène-1 et/ou un mélange de ces produits. De préférence, l'initiateur à base de produit chloré résulte de la chloration additive d'hexachlorobutadiène-1,3.

L'invention concerne également un initiateur à base de produit chloré pour la déshydrochloration pyrolytique d'halogénoalcanes qui résulte de la chloration additive d'hexachlorobutadiène-1,3 et qui comprend principalement du décachlorobutane et/ou un octachlorobutène tel que l'octachlorobutène-1 suivant le degré auquel la chloration additive a été poussée.

Par produit chloré, on entend le ou les produits résultant de la chloration additive d'hexachlorobutadiène-1,3, pouvant être obtenu de manière connue en soi, par exemple par photochloration ou par chloration en phase liquide catalysée par le fer. Quand la chloration additive de l'hexachlorobutadiène conduit à la formation de mélanges de produits, ces mélanges contiennent, en plus des produits principaux cités plus haut, un peu d'hexachloréthane et éventuellement de l'hexachlorobutadiène-1,3 non transformé, ainsi qu'une faible proportion de divers autres produits. Quand la chloration additive de l'hexachlorobutadiène conduit à la formation d'un produit chloré unique, il s'agit généralement d'un octachlorobutène tel que l'octachlorobutène-1 ou du décachlorobutane.

L'obtention d'un mélange de produits ou d'un produit chloré unique dépend notamment du degré de chloration de l'hexachlorobutadiène, de la température et de la nature du catalyseur.

Il est toutefois évident que par mélange de produits chlorés résultant de la chloration additive d'hexachlorobutadiène-1,3, on entend non seulement les mélanges issus directement de la chloration mais également les mélanges ayant, après la chloration, subi certaines modifications, tels que celles résultant d'opérations de distillation ou de rectification ainsi que d'opérations de cristallisation, permettant d'obtenir un enrichissement en l'un ou l'autre des constituants du mélange.

Enfin en ce qui concerne le produit chloré unique issu généralement de la chloration additive de l'hexachlorobutadiène-1,3 pouvant être mis en oeuvre dans le procédé de l'invention, il est également évident que leur origine n'est pas critique en soi pour le procédé de déshydrohalogénation pyrolytique de l'invention et que ce produit unique peut avoir comme origine tout autre matière première que l'hexachlorobutadiène-1,3.

A titre d'exemple, on donne ci-après la composition, en g/kg, de deux mélanges bruts résultant de la photochloration de l'hexachlorbutadiène-1,3 tel que décrit à l'exemple 2 ci-après:

2

0 172 596

|  | Mélange A | Mélange B |
|---|---|---|
| Octachlorobutène-1 | 415 | - |
| Décachlorobutane | 303 | 812 |
| Hexachloréthane | 183 | 147 |
| Hexachlorobutadiène-1,3 | 77 | < 0,5 |
| Autres produits, non identifiés | 22 | 41 |

Ces mélanges bruts de produits ainsi que d'ailleurs certains constituants de ces mélanges peuvent être utilisés dans le procédé selon l'invention, en une quantité pondérale comprise habituellement entre 0,01 et 10 % et de préférence entre 0,1 et 5 % par rapport à l'halogénoalcane soumis à la déshydrochloration pyrolytique. De bons résultats ont été obtenus avec des quantités pondérales, du Mélange A et du Mélange B, voisines de 1 % par rapport à l'halogénoalcane soumis à la déshydrochloration pyrolytique. On a également obtenu de bons résultats en mettant en oeuvre respectivement 1 % en poids d'octachlorobutène-1 et 1 % en poids de décachlorobutane par rapport au poids de l'halogénoalcane.

Les initiateurs à base de produit chloré selon l'invention ont un effet accélérateur très puissant dans des zones de température bien inférieure à celles qui sont nécessaires quand on utilise les initiateurs connus. Ainsi dans le cas de la pyrolyse du 1,2-di-chloréthane gazeux à la pression atmosphérique, on obtient déjà des taux de pyrolyse intéressants avec les mélanges bruts de la chloration additive de l'hexachlorbutadiène-1,3 mis en oeuvre à raison de 1 % en poids par rapport au 1,2-dichloréthane à des températures de 300 à 425°C. Ces taux de pyrolyse augmentent bien entendu avec l'élévation de la température mais au prix de l'apparition de réactions secondaires qui donnent lieu à la formation de sous-produits indésirables. Le procédé selon l'invention permet donc de choisir une solution de compromis au niveau de la température de la pyrolyse.

Il résulte des travaux publiés par P.J.Thomas, dans Current Topics in Mass Spectrometry, Chem. Kinet. Proc. Symp. 1981, pages 115 à 140 (Chemical Abstracts 97, 197708u) que, dans le cas de la pyrolyse du 1,2-dichloréthane, les taux de formation des principaux sous-produits tels que l'acétylène, le 1-chlorobutadiène-1,3 et le 2-chlorobutadiène-1,3 par rapport au chlorure de vinyle produit, augmentent avec le taux de pyrolyse d'une part et avec la température d'autre part. Dès lors, les principaux avantages que procure le procédé selon l'invention, par rapport au procédé non initié peuvent se résumer comme suit:

1. A taux de pyrolyse et production donnés, on peut réduire la temperature de la déshydrochloration pyrolytique (d'où une économie d'énergie) et améliorer la sélectivité et donc le rendement.

2. A température et production données, on peut travailler avec un taux de pyrolyse plus élevé, ce qui réduit la proportion d'halogénoalcanes à recycler et procure de ce fait une économie d'énergie.

3. A température et taux de pyrolyse données, on peut augmenter la production et la productivité.

4. Les avantages susmentionnés peuvent être combinés; par exemple, à production constante, mais à température réduite et à taux de pyrolyse augmenté, on peut réaliser une économie d'énergie sans perte de sélectivité.

Un avantage important du procédé selon l'invention réside dès lors dans le fait qu'il peut être appliqué à des températures inférieures à celles que l'on peut utiliser dans les procédés de l'art antérieur. Des températures comprises entre 200 et 450°C ont donné de bons résultats pour les différents halogénoalcanes étudiés.

Un autre avantage du procédé selon l'invention réside dans le fait qu'il peut être exploité soit à la pression atmosphérique, soit sous une pression supérieure. De préférence on opère sous pression atmosphérique ou sous une pression modérée.

Par pression modérée, on entend des pressions inférieures à 20 atmosphères (20,4 bars). Des bons résultats ont été obtenus à des pressions comprises entre 1 et 15 bars.

Le procédé de déshydrochloration pyrolytique d'halogénoalcanes selon l'invention peut être appliqué à un grand nombre de réactions. On peut notamment, sans aucune intention limitative, mentionner les applications suivantes:

- fabrication de chlorure de vinyle à partir de 1,2-dichloréthane
- fabrication de chlorure de vinylidène et de 1,2-dichloréthylène cis et trans à partir de 1,1,2-trichloréthane
- fabrication de trichloréthylène à partir de 1,1,2,2-tétrachloréthane
- fabrication de fluorure de vinylidène à partir de 1-chloro-1,1-difluoréthane.

Le procédé selon l'invention peut aussi être appliqué lorsque la déshydrochloration pyrolytique de l'halogénoalcane est combinée avec une chloration substitutive du produit issu de la pyrolyse, c'est-à-dire dans le cas d'une pyrolyse chlorée. C'est ainsi que le procédé selon l'invention trouve notamment une utilisation pour la fabrication de trichloréthylène et de perchloréthylène par pyrolyse chlorée de 1,2-dichloréthane seul ou en mélange avec d'autres hydrocarbures chlorés.

On a également constaté qu'il peut être souhaitable dans certains cas, afin de minimiser la surchauffe du mélange réactionnel, d'exécuter la réaction de déshydrochloration pyrolytique en présence d'additifs qui agissent comme diluants mais qui sont inertes vis-à-vis des réactifs et des initiateurs intervenant dans la réaction. Comme additifs on utilise de préférence des dérivés chlorés aliphatiques tel que le tétrachlorure de carbone ou des produits inorganiques tels que le chlorure d'hydrogène ou l'azote. De préférence on opère avec le tétrachlorure de carbone.

En général les additifs organiques halogénés sont ajoutés au milieu réactionnel à raison de 1 à 25 moles par mole d'halogénoalcane mis en oeuvre.

Le procédé selon l'invention peut être réalisé dans tout appareillage ou tout réacteur permettant de réunir

3

les conditions opératoires décrites ci-avant.

Les exemples suivants sont donnés à titre explicatif du procédé selon l'invention.

**Exemple 1**

On a effectué une série d'essais qui illustrent l'efficacité des produits de la chloration additive de l'hexachlorbutadiène-1,3, à savoir respectivement les mélanges bruts A et B mentionnés plus haut, l'octachlorobutène-1 et le décachlorobutane, en tant qu'initiateurs pour la déshydrochloration pyrolytique du 1,2-dichloréthane. Ces essais ont été réalisés à la pression atmosphérique dans un réacteur tubulaire en quartz carbonacé d'une longueur de 260 mm et d'un diamètre intérieur de 12,2 mm, précédé d'un préchauffeur coaxial également en quartz carbonacé d'une longueur de 170 mm et d'un diamètre intérieur de 8,1 mm, l'ensemble contenant sur toute la longueur une gaine axiale pour thermocouples d'un diamètre extérieur de 6 mm (volume du réacteur=23 cm$^3$; volume du préchauffeur =2,0 cm$^3$). Des résistances électriques bobinées sur le réacteur permettent de maintenir un profil de température uniforme tout le long de celui-ci. On alimente le réacteur en 1,2-dichloréthane liquide (pureté > 99,98 %), à un débit constant et connu, à l'aide d'une pompe doseuse. Le dichloréthane est vaporisé, avant d'entrer dans le réacteur, dans le préchauffeur présentant un gradient de température entre la température ambiante et la température réactionnelle. Dans le cas des essais réalisés en présence d'un initiateur de pyrolyse, celui-ci est préalablement dissous dans le 1,2-dichloréthane. Le mélange gazeux sortant du réacteur est dilué par un courant d'azote, afin d'éviter la condensation de certains constituants, débarrassé de son contenu en HCl par barbottage dans de l'eau et soumis à une analyse par chromatographie en phase vapeur afin de déterminer le taux de pyrolyse. On a mesuré le taux de pyrolyse atteint, à différentes températures, avec les initiateurs à base de produit chloré selon l'invention et à titre comparatif avec les initiateurs de l'art antérieur et en l'absence d'initiateurs, le temps de séjour (volume du four divisé par le débit gazeux d'alimentation, à la température de l'essai) étant maintenu à peu près constant. Les résultats de ces essais sont rassemblés au tableau 1 ci-après dans lequel on a groupé les essais par zones de température de déshydrochloration pyrolytique. Ils montrent clairement l'effet accélérateur très puissant des initiateurs à base de produit chloré selon l'invention, dans une mesure qui les rend surtout intéressants quand on les met en oeuvre à basse température par exemple entre 200 et 450°C et dans le cas où la déshydrochloration pyrolytique du 1,2-dichloroéthane est effectuée en phase gazeuse lorsqu'on les utilise entre 300 et 425°C.

Comme initiateurs de l'art antérieur, on a utilisé le tétrachlorure de carbone, le perchloréthyléne, l'hexachloréthane et comme autre essai de référence, on a utilisé un mélange synthétique représentant les impuretés (produits autres que l'octachlorobutène-1 et le décachlorobutane) présentes dans le mélange A. Les numéros des essais comparatifs du tableau 1 sont suivis de la lettre R.

On remarque que le tétrachlorure de carbone, le perchloréthylene et l'hexachloréthane utilisés isolément aux températures auxquelles les initiateurs à base de produit chloré selon l'invention sont déjà très efficaces (300°C) ne donnent aucun résultat tangible. A partir de 350°C, l'hexachloréthane acquiert une certaine efficacité, mais qui reste inférieure à celle des mélanges A ou B, et il présente, en outre, l'inconvénient de conduire à la formation de sous-produits à trois atomes de carbone. Quant au mélange synthétique représentant les impuretés présentes dans le mélange A, il reste lui aussi peu efficace, même à 425°C.

**TABLEAU 1**

| N° d'essai | Température, °C | Temps de séjour, s | Initiateur | Taux de pyrolyse, % | Commentaires |
|---|---|---|---|---|---|
| 1 | 302 | 10,0 | 1 % poids de mèlange A | 43,2 | |
| 2 | 304 | 9,9 | 1 % poids de melange B | 45,4 | |
| 3 | 304 | 10,3 | 1 % poids d'octachloro-butène-1* | 32,4 | |
| 4 | 300 | 10,0 | 1 % poids de décachloro-butane* | 39,8 | - |
| 5R | 303 | 9,9 | 1 % poids de $C_2Cl_6$ | 0,7 | |
| 6R | 300 | 10,1 | 1 % poids de $CCl_4$ | 0,1 | |
| 7R | 349 | 10,5 | - | 1,8 | |
| 8 | 351 | 9,9 | 1 % poids de mélange A | 68,4 | |
| 9 | 350 | 10,6 | 1 % poids de mélange B | 60,5 | |
| 10 | 355 | 10,5 | 1 % poids d'octachloro-butène-1 | 72,1 | - |
| 11 | 355 | 9,6 | 1 % poids de décachloro-butane | 59,3 | |
| 12R | 354 | 10,6 | 1 % poids de $C_2Cl_6$ | 32,2 | |
| 13R | 350 | 10,9 | 1 % poids de $CCl_4$ | 1,9 | |
| 14R | 350 | 10,1 | 0,18 % poids de $C_2Cl_6$ 0,08 % poids de $C_4Cl_6$ | 3,3 | Il s'agit d'un mélange syn-thétique représentant 1 % de m'elange A <u>moins</u> son contenu de $C_4Cl_{10}$ et $C_4Cl_8$ (Impu-retés mélange A). |
| 15R | 423 | 8,4 | - | 14,1 | Résultates peu reproductibles dus probablement à des effets de paroi. |
| 16R | 425 | 10,0 | - | 6,2 | |
| 17 | 427 | 9,6 | 1 % poids de mélange A | 82,5 | |
| 18 | 422 | 10,0 | 1 % poids de mélange B | 74,5 | |
| 19 | 426 | 10,1 | 1 % poids d'octachloro-butène-1 | 85,4 | |
| 20 | 433 | 10,2 | 1 % poids de décachloro-butane | 76,3 | |
| 21R | 426 | 10,2 | 1 % poids de $C_2Cl_6$ | 65,4 | |
| 22R | 427 | 10,8 | 0,18 % poids de $C_2Cl_6$ 0,08 % poids de $C_4Cl_6$ | 19,2 | Mélange synthétique comme à l'essai n°14R (Impuretés |
| 23R | 431 | 10,1 | 1 % poids de $C_2Cl_4$ | 15,8 | |
| 24R | 529 | 8,1 | - | 64,3 | |
| 25 | 529 | 8,1 | 1 % poids de mélange A | 98,5 | |

* Puretés de l'octachlorobut*ene-1 et du décachlorobutane > 99 %.

## Exemple 2

Dans cet exemple on décrit le procédé utilisé pour la photochloration d'hexachlorobutadiène-1,3 en vue d'obtenir les mélanges bruts utilisables comme initiateurs des réactions de déshydrochloration pyrolytique d'halogénoalcanes.

Le réacteur de photochloration, en verre pyrex, est constitué de trois tubes concentriques verticaux. La lampe actinique (tube fluorescent Philips TL05, 120 W) est placée axialement, à l'intérieur du tube de plus petit diamètre. L'espace annulaire, fermé aux extrémités, entre celui-ci et le deuxième tube, constitue la zone de réaction. Dans l'espace annulaire entre les deuxième et troisième tubes, également fermé aux extrémités, une circulation de fluide réfrigérant permet de maintenir la température de la zone de réaction à 3°C (un courant d'azote balaye l'espace entre la lampe et le réacteur, afin d'éviter la condensation d'humidité atmosphérique). Les diamètres intérieur et extérieur et la hauteur de la zone de réaction sont respectivement de 55, 78 mm et 210 mm, ce qui correspond à un volume de 505 $cm^3$. Dans le bas du réacteur, un injecteur en forme de couronne perforée permet l'introduction de chlore gazeux. Le réacteur est également équipé d'un robinet de vidange et d'une tubulure reliant le ciel du réacteur, via un condenseur à reflux, à un barboteur contenant de la soude caustique.

On introduit dans le réacteur 600 g (3,90 mol) de tétrachlorure de carbone et 200 g (0,77 mol) d'hexachlorobutadiène-1,3. On introduit du chlore gazeux à un débit d'environ 2,5 litres normaux par heure.

Après 26 h, on soutire le produit et on le lave à la soude caustique 2N (2 x 400 cm³), puis à l'eau (5 x 400 cm³). Après élimination du CCl₄ à l'évaporateur rotatif, à 80°C et sous pression réduite (5 torr), on obtient 230 g de mélange A.

Si on prolonge la durée de la photochloration jusqu'à 56 h, on obtient, après lavage du produit et élimination du CCl₄, 200 g de mélange B.

## Exemple 3

On opère comme à exemple 1 mais en mettant en oeuvre du 1,1,2,2-tétrachloréthane en vue de fabriquer du trichloréthylène. Les résultats obtenus sont récapitulés au tableau 2 ci-après.

**TABLEAU 2**

| Température, °C | Temps de séjour, s | Initiateur | Taux de pyrolyse, %* |
|---|---|---|---|
| 351 | 10,5 | - | 4,3 |
| 353 | 10,1 | 1 % poids de mélange A | 75,8 |
| 304 | 12,3 | 1 % poids de mélange A | 61,6 |

* calculé à partir des débits de 1,1,2,2-tétrachloréthane alimenté et de chlorure d'hydrogène formé.

## Exemple 4

On opère comme à l'exemple 1 mais en mettant en oeuvre du 1,1,2-trichloréthane en vue de fabriquer des dichloréthylènes. Les résultats obtenus sont récapitulés au tableau 3 ci-après.

**TABLEAU 3**

| Température, °C | Temps de séjour, s | Initiateur | Taux de pyrolyse, %** |
|---|---|---|---|
| 424 | 11,6 | - | 2,7 |
| 424 | 10,3 | 1 % poids de mélange A | 36,7 |

** calculé à partir des débits de 1,1,2-trichloréthane alimenté et de chlorure d'hydrogène formé.

## Exemple 5

Dans cet exemple, on décrit le procédé utilisé pour la chloration en phase liquide catalysée par le fer d'hexachlorobutadiène-1,3 en vue d'obtenir des mélanges bruts utilisables comme initiateurs des réactions de déshydrochloration pyrolytique d'halogénoalcanes.

Dans un autoclave agité en acier inoxydable d'un volume de 1,5 dm³ équipé d'un chauffage par circulation d'huile dans une double enveloppe on introduit:

- 1190 g (4,60 mol) d'hexachlorobutadiène-1,3 de pureté supérieure à 98 % en poids,
- 1,34 g de FeCl₃ anhydre.

Après avoir porté le contenu du réacteur à 125°C, on introduit graduellement du chlore liquide, à partir d'un réservoir mesureur maintenu sous pression d'azote. Le débit d'introduction du chlore est ajusté de manière à maintenir dans le réacteur une pression absolue de 9 bars. La réaction est arrêtée après 10 h, lorsqu'on a introduit 652 g (9,20 mol) de chlore.

Le milieu réactionnel est strippé à l'azote pour éliminer le chlore résiduaire, puis lavé avec HCl 6N jusqu'à élimination du FeCl₃. Le produit obtenu présente la composition suivante en g/kg:

**0 172 596**

**Mélange C**

| | |
|---|---|
| octachlorobutène-1 | 200 |
| décachlorobutane | 615 |
| hexachloréthane | 128 |
| hexachlorobutadiène-1,3 | 25 |
| autres produits, non identifiés | 32 |

**Exemple 6**

Dans cet exemple on décrit un procédé de déshydrochloration pyrolytique du 1,2-dichloréthane et de ses produits de chloration substitutive. Ce procédé est également appelé pyrolyse chlorée du 1,2-dichloréthane. L'initiateur mis en oeuvre dans cet exemple est le mélange C décrit dans l'exemple 5 ci-dessus.

La pyrolyse chlorée du 1,2-dichloréthane en phase gazeuse a été effectuée à la pression atmosphérique dans un réacteur mélangeur continu sphérique d'environ 1 dm³, en pyrex, auto-agité par jets gazeux (Cf. Chem. Eng. Sci., 1973, 28, p.129-137), les réactifs étant introduits sous forme gazeuse à l'aide d'un injecteur à quatre tuyères placé au milieu de la sphère. Le réacteur est placé dans une enceinte à l'intérieur de laquelle l'air est chauffé électriquement et brassé à l'aide d'une turbine, afin de maintenir la température réactionnelle désirée. Le 1,2-dichloréthane et le diluant (CCl₄) destiné à modérer l'effet thermique de la réaction, sont alimentés via un évaporateur tubulaire vertical, chauffé électriquement et raccordé à l'injecteur du réacteur. Le chlore gazeux est injecté dans le pied du tube évaporateur. L'initiateur, dans le cas où on l'a utilisé, a été ajouté sous forme liquide, en solution très concentrée dans du CCl₄, par une dérivation donnant dans le conduit d'introduction des réactifs vaporisés. Les produits de pyrolyse-chloration sortent du réacteur par une tubulure diamétralement opposée à l'introduction, puis ils sont condensés, traités par du NaOH aqueux pour neutraliser le chlore résiduaire et le HCl formé; après décantation, la phase organique est séparée de la phase aqueuse et analysée par chromatographie en phase vapeur. Les conditions et les résultats des essais sont donnés au tableau 4 ci-après.

**TABLEAU 4**

|  |  | Essai de reference, sans initiateur | Essai avec initiateur |
|---|---|---|---|
| **Conditions** |  |  |  |
| - température dans le réacteur | °C | 302 | 301 |
| -température sortie évaporateur $(1,2\text{-}C_2H_4Cl_2 + CCl_4 + Cl_2$ | °C | 143 | 145 |
| - introduction initiateur |  | néant | solution à 38 % poids dans $CCl_4$ |
| - temps de séjour moyen* | s | 10 | 10 |
| - rapports molaires des réactifs |  |  |  |
| . 1,2-dichloréthane | mol/mol | 1 | 1 |
| . $Cl_2$ | mol/mol | 1,8 | 1,8 |
| . $CCl_4$ (diluant inerte) | mol/mol | 2,5 | 2,5 |
| . initiateur (somme de ses constituants) | mol/mol | 0 | 0,05 |
| - teneur des réactifs en initiateur | % mol | 0 | 1,0 |
| **Resultats** |  |  |  |
| - répartition des produits de chloration |  |  |  |
| .1,2-dichloréthane | % mol | 22,2 | < 0,1 |
| .1,2-dichloréthylène cis et trans | % mol | 1,0 | 28,8 |
| . 1,1,2-trichloréthane | % mol | 40,8 | 4,0 |
| .trichloréthylène | % mol | 1,2 | 39,3 |
| . 1,1,1,2-tétrachloréthane | % mol | 14,0 | 9,5 |
| . 1,1,2,2-tétrachloréthane | % mol | 16,8 | 6,8 |
| . perchloréthylène | % mol | < 0,1 | 5,9 |
| . pentachloréthane | % mol | 3,9 | 5,6 |
| - conversion du $1,2\text{-}C_2H_4Cl_2$ | % | 78 | >99,9 |
| - taux de chloration** | mol/mol | 1,18 | 1,79 |
| - taux de déshydrochloration*** | mol/mol | 0,02 | 0,74 |

$$* \text{ temps de séjour moyen} = \frac{\text{volume du réacteur}}{\text{débit volumique des réactifs (à la température réactionnelle)}}$$

$$** \text{ taux de chloration} = \frac{\text{moles de chlore ayant réagi avec le 1, 2-dichloréthane}}{\text{moles de 1,2-dichloréthane mis en oeuvre}}$$

$$*** \text{ taux de déshydrochloration} = \frac{\text{moles composés insaturés}}{\text{moles totalité des composés à 2 atomes de carbone}}$$

On peut déduire des résultats du tableau 4 que vers 300°C, il y a une chloration relativement importante <u>sans initiateur.</u>

Les principaux produits sont des composés saturés tels que le 1,1,2-trichloréthane, les 1,1,1,2- et 1,1,2,2-tétrachloréthanes.

L'injection de 1 % mol <u>d'initiateur</u> entraîne les effets suivants:

- augmentation du taux de chloration de 1,18 à 1,79 ce qui correspond à une consommation quasi-totale du chlore;

- augmentation du taux de déshydrochloration de 0,02 à 0,74 mol/mol, c'est-à-dire les produits principaux sont des insaturés: 1,2-dichloréthylène cis et trans, trichloréthylène.


**Exemple 7**

Dans cet exemple on effectue une série d'essais de déshydrochloration pyrolytique du 1,2-dichloréthane sous pression afin d'illustrer l'action initiatrice des produits de chloration de l'hexachlorobutadène.

Ces essais ont été réalisés sous 12 atmosphères dans un réacteur tubulaire en acier inoxydable carbonacé d'une longueur de 320 mm et d'un diamètre intérieur de 20 mm. Le réacteur contient une gaine pour thermocouple de 10 mm de diamètre extérieur située au centre du réacteur.

Le volume utile du réacteur est de 7,5 cm$^3$. Le préchauffage du réactif de la température ambiante jusqu'à la température de réaction souhaitée ainsi que sa vaporisation sont assurés par un réchauffeur de 10 cm$^3$ de volume, monté à l'entrée du réacteur.

Des résistances électriques dont les puissances peuvent être ajustées assurent un chauffage uniforme au long du réacteur. Le réacteur est alimenté en 1,2-dichloréthane pur (> 99,98 %), contenant soit un initiateur [mélange C (essais 1, 2, 3, 4, 8, 9)] à l'état dissous, au moyen d'une pompe doseuse délivrant un débit constant et connu soit sans initiateur (essais 5, 6 et 8).

Le maintien à pression constante du réacteur et la détente à pression atmosphérique des gaz quittant la zone réactionnelle sont assurés par une vanne de régulation automatique.

L'HCl produit est abattu dans un scrubber arrosé par de l'eau. On retrouve au pied de ce scrubber une grande partie du 1,2-dichloréthane non converti. Les gaz non condensés sont principalement composé de chlorure de vinyle.

Les phase organiques liquides ou gazeuses sont analysées par chromatographie en phase vapeur. L'HCl produit est titré. Ces dosages permettent d'établir les taux de pyrolyse atteints à différentes températures, avec les initiateurs à base de produit chloré selon l'invention et à titre comparatif en l'absence d'initiateur.

Les résultats obtenus sont rassemblés au tableau 5. Le temps de séjour mentionné correspond au volume du four divisé par le volume gazeux des réactifs à la température et à la pression de l'essai. Ceux-ci sont classés par ordre croissant de température.

Tout comme lors des essais à pression atmosphérique décrit à l'exemple 1, on note une accélération remarquable de la réaction en présence des produits de chloration de l'hexachlorobutadiène-1,3; des taux de pyrolyse proches de 50 % sont obtenus entre 350 et 375°C, alors qu'en l'absence d'initiateur (essais dont les numéros sont suivis de la lettre R), il faut atteindre 480°C pour obtenir un taux de pyrolyse comparable.

**TABLEAU 5**

| N° d'essai | Température °C | Temps de séjour, s | Initiateur % poids | Taux de pyrolyse, % |
|---|---|---|---|---|
| 1 | 323 | 10,0 | 1 % mélange C | 37 |
| 2 | 349 | 10,0 | " | 48 |
| 3 | 375 | 10,0 | " | 54 |
| 4 | 400 | 10,1 | " | 55 |
| 5 | 400 | 10,0 | " | 13 |
| 6R | 439 | 10,0 | - | 19 |
| 7 | 452 | 10,1 | 1 % mélange C | 67 |
| 8R | 480 | 11 | - | 53 |

## Revendications

1. Procédé de déshydrochloration pyrolytique d'halogénoalcanes en présence d'un initiateur à base de produit chloré caractérisé en ce qu'il comprend principalement du décachlorobutane ou un octachlorobutène tel que l'octachlorobutène-1 ou un mélange de ces produits.

2. Procédé suivant la revendication 1 caractérisé en ce que l'initiateur à base de produit chloré résulte de la chloration additive de l'hexachlorobutadiène-1,3.

3. Procédé suivant les revendications 1 ou 2 caractérisé en ce qu'on effectue la déshydrochloration à une température supérieure à 200°C.

4. Procédé suivant les revendications 1 à 3 caractérisé en ce qu'on utilise l'initiateur en une quantité de l'ordre de 1 % du poids de l'halogénoalcane mis en oeuvre.

5. Procédé suivant l'une des revendications 1 à 4 caractérisé en ce qu'il est appliqué à la fabrication de chlorure de vinyle par déshydrochloration pyrolytique de 1,2-dichloréthane.

6. Procédé suivant l'une des revendications 1 à 4 caractérisé en ce qu'il est appliqué à la fabrication de chlorure de vinylidène par déshydrochloration pyrolytique de 1,1,2-trichloréthane.

7. Procédé suivant l'une des revendications 1 à 4 caractérisé en ce qu'il est appliqué à la fabrication de trichloréthylène par déshydrochloration pyrolytique de 1,1,2,2-tétrachloréthane ou de 1,1,1,2-tétrachloréthane.

8. Procédé suivant l'une des revendications 1 à 4 caractérisé en ce qu'il est appliqué à la fabrication de fluorure de vinylidène par déshydrochloration pyrolytique de 1-chloro-1,1-difluoroéthane.

9. Procédé suivant l'une des revendications 1 à 4 caractérisé en ce qu'il est appliqué à la fabrication de trichloréthylène et de perchloréthylène par pyrolyse chlorée de 1,2-dichloréthane.

10. Initiateur à base de produit chloré pour la déshydrochloration pyrolytique d'halogénoalcanes caractérisé en ce qu'il résulte de la chloration additive d'hexachlorobutadiène-1,3 et qu'il comprend principalement du décachlorobutane et/ou un octachlorobutène tel que l'octachlorobutène-1.

## Patentansprüche

1. Verfahren zur pyrolytischen Dehydrochlorierung von Halogenalkanen in Anwesenheit eines Initiators auf Basis eines chlorierten Produktes, dadurch gekennzeichnet, daß dieses im wesentlichen besteht aus Dekachlorbutan oder einem Oktachlorbuten wie dem Oktachlorbuten-1 oder einer Mischung dieser Produkte.

2. Verfahren gemäß Anspruch 1, dadurch gekennzeichnet, daß der Initiator auf Basis eines chlorierten Produktes herstammt aus der Additionschlorierung von Hexachlorbutadien-1,3.

3. Verfahren gemäß Anspruch 1 oder 2, dadurch gekennzeichnet, daß man die Dehydrochlorierung bei einer Temperatur oberhalb von 200°C durchführt.

4. Verfahren gemäß einem der Ansprüche 1 bis 3, dadurch gekennzeichnet, daß man Initiator in einer Menge in der Größenordnung von 1 Gew.-% bezogen auf eingesetztes Halogenalkan verwendet.

5. Verfahren gemäß einem der Ansprüche 1 bis 4, dadurch gekennzeichnet, daß es angewandt wird auf die Herstellung von Vinylchlorid durch pyrolytische Dehydrochlorierung von 1,2-Dichlorethan.

6. Verfahren gemäß einem der Ansprüche 1 bis 4, dadurch gekennzeichnet, daß es angewandt wird auf die Herstellung von Vinylidenchlorid durch pyrolytische Dehydrochlorierung von 1,1,2-Trichlorethan.

7. Verfahren gemäß einem der Ansprüche 1 bis 4, dadurch gekennzeichnet, daß es angewandt wird auf die Herstellung von Trichlorethylen durch pyrolytische Dehydrochlorierung von 1,1,2,2-Tetrachlorethan oder von 1,1,1,2-Tetrachlorethan.

8. Verfahren gemäß einem der Ansprüche 1 bis 4, dadurch gekennzeichnet, daß es angewandt wird auf die Herstellung von Vinylidenfluorid durch pyrolytische Dehydrochlorierung von 1-Chlor-1,1-difluorethan.

9. Verfahren gemäß einem der Ansprüche 1 bis 4, dadurch gekennzeichnet, daß es angewandt wird auf die Herstellung von Trichlorethylen und Perchlorethylen durch chlorierende Pyrolyse von 1,2-Dichlorethan.

10. Initiator auf der Basis eines chlorierten Produktes für die pyrolytische Dehydrochlorierung von Halogenalkanen dadurch gekennzeichnet, daß er herstammt aus der Additionschlorierung von Hexachlorbutadien-1,3 und daß er im wesentlichen besteht aus Dekachlorbutan und/oder einem Oktachlorbuten wie Oktachlorbuten-1.

## Claims

1. Process for the pyrolytic dehydrochlorination of haloalkanes in the presence of an initiator based on chlorinated product, characterized in that it comprises chiefly decachlorobutane or an octachlorobutene such as octachloro-1-butene or a mixture of these products.

2. Process according to Claim 1, characterized in that the initiator based on chlorinated product results from the additive chlorination of hexachloro-1,3-butadiene.

3. Process according to Claims 1 or 2, characterized in that the dehydrochlorination is carried out at a temperature above 200°C.

4. Process according to Claims 1 to 3, characterized in that the initiator is used in a quantity of the order of 1 % of the weight of the haloalkane employed.

5. Process according to one of Claims 1 to 4, characterized in that it is applied to the manufacture of vinyl chloride by pyrolytic dehydrochlorination of 1,2-dichloroethane.

6. Process according to one of Claims 1 to 4, characterized in that it is applied to the manufacture of vinylidene chloride by pyrolytic dehydrochlorination of 1,1,2-trichloroethane.

7. Process according to one of Claims 1 to 4, characterized in that it is applied to the manufacture of trichloroethylene by pyrolytic dehydrochlorination of 1,1,2,2-tetrachloroethane or of 1,1,1,2-tetrachloroethane.

8. Process according to one of Claims 1 to 4, characterized in that it is applied to the manufacture of vinylidene fluoride by pyrolytic dehydrochlorination of 1-chloro-1,1-difluoroethane.

9. Process according to one of Claims 1 to 4, characterized in that it is applied to the manufacture of trichloroethylene and of perchloroethylene by chlorination pyrolysis of 1,2-dichloroethane.

10. Initiator based on chlorinated product for the pyrolytic dehydrochlorination of haloalkanes, characterized in that it is produced by the additive chlorination of hexachloro-1,3-butadiene and that it comprises chiefly decachlorobutane and/or an octachlorobutene such as octachloro-1-butene.